(19) Europäisches Patentamt
European Patent Office
Office européen des brevets

(11) Publication number: 0 002 077 B1

(12) # EUROPEAN PATENT SPECIFICATION

(45) Date of publication of patent specification: 02.09.81

(51) Int. Cl.³: C 07 C 69/74, C 07 C 67/22

(21) Application number: 78200248.9

(22) Date of filing: 17.10.78

(54) **Esters of substituted cyclopropanecarboxylic acids and process for the preparation thereof.**

(30) Priority: 27.10.77 GB 4477477

(43) Date of publication of application:
30.05.79 Bulletin 79/11

(45) Publication of the grant of the European patent:
02.09.81 Bulletin 81/35

(84) Designated Contracting States:
BE CH DE FR GB LU NL

(56) References cited:
DE - A - 2 751 610
GB - A - 1 420 636

Chemical Abstracts vol 88, no. 13, 1978 "3-(2,2-Dichlorovinyl)-2,2-dimethylcyclopropanecarbonitrile" page 481, column 1, abstract no. 89 201d

(73) Proprietor: SHELL INTERNATIONALE RESEARCH MAATSCHAPPIJ B.V.
Carel van Bylandtlaan 30
NL-2596 HR DEN HAAG (NL)

(72) Inventor: Syrier, Johannes Leopold Marie
Badhuisweg 3
NL-1031 CM Amsterdam (NL)

(74) Representative: Keuzenkamp, Abraham et al,
P.O. Box 302
NL-2501 CH Den Haag (NL)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 002 077 B1

# Esters of substituted cyclopropanecarboxylic acids and process for the preparation thereof

This invention relates to a process for the preparation of esters of substituted cyclopropanecarboxylic acids and to the novel esters produced thereby.

Substituted cyclopropanecarboxylic acids are useful intermediates in the preparation of pesticidally-active esters thereof, especially the 3-phenoxybenzyl and *alpha*-cyano-3-phenoxybenzyl esters; these pesticidally active compounds, the so-called "synthetic pyrethroids", have exceptionally good insecticidal properties whilst possessing a very low mammalian toxicity, see British Patent Specifications 1,413,491 and 1,446,304. This combination of properties makes them of considerable interest to the agrochemical industry and much effort has been expended in determining economic routes to these "synthetic pyrethroids", especially to the manufacture of substituted cyclopropanecarboxylic acids which represent the portion of the pyrethroid compound having no established synthetic routes available in the literature.

In a search for economic routes to the substituted cyclopropanecarboxylic acids the Applicant has successfully managed to synthesize the substituted cyclopropanecarbonitrile but hitherto has been unable to convert satisfactorily the nitrile into the corresponding acid or ester.

The Applicant has attempted alcoholysis in the presence of an acid catalyst according to the many recommendations in standard text books but found that the yields of the corresponding alkyl ester were very poor and that the reactions often resulted in the production of large quantities of lactones and other by-products.

It is also known to convert nitriles into esters by means of the Pinner Reaction (see "Houben-Weyl Handbuch der Organischen Chemie", H. Heneka, Book 8, pages 536—539 (1952) and "Chemical Reviews", Robert Roger and Douglas Neilson, pages 181—184 (1961)), involving the action of an alkanol and hydrogen bromide or hydrogen chloride on a nitrile at ambient temperature or lower temperatures under anhydrous conditions and hydrolyzing the resulting water-sensitive imino-ether. In the case of cyclopropanecarbonitriles, however, the Pinner Reaction conditions had no effect even after a period of 24 hours.

Surprisingly, the Applicant has now found that replacement of the alkanol by two specific alkoxyalkanols and a modification of the Pinner conditions has resulted in a very high conversion of a substituted cyclopropanecarbonitrile into the alkoxyalkyl ester of the corresponding substituted cyclopropanecarboxylic acid.

The present invention, therefore, provides a process for the manufacture of an ester having the following general formula:

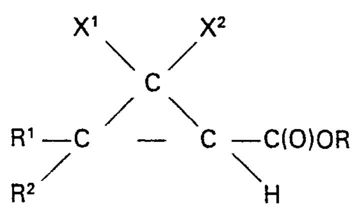
(I)

(wherein

each of $R^1$ and $R^2$ independently represents an alkyl group of 1 to 4 carbon atoms or $R^1$ and $R^2$ together represent an alkylene group of up to 5 carbon atoms, other than methylene, each of $X^1$ and $X^2$ independently represents a hydrogen atom, an alkyl group of 1 to 4 carbon atoms, a halogen atom, or $X^1$ and $X^2$ together represent an alkylene group of up to 5 carbon atoms, other than methylene, or $X^1$ represents a hydrogen atom and $X^2$ a 2-halovinyl group, a 2,2-dihalovinyl group or a 2-methyl-1-propenyl group, and R represents a 2-methoxyethyl or a 2-ethoxyethyl group), which process comprises reacting a substituted cyclopropanecarbonitrile having the general formula:—

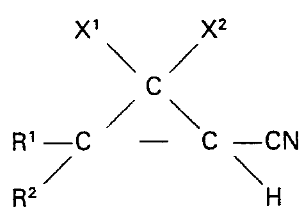
(II)

wherein

$R^1$, $R^2$, $X^1$ and $X^2$ have the same meaning as in formula I, with 2-methoxyethanol or 2-ethoxyethanol under substantially anhydrous conditions and in the presence of mineral acid acting as reaction catalyst and as scavenger for ammonia produced during the reaction.

Preferably, the reaction temperature in the process according to the invention is maintained in the range 80°C to 200°C and particularly in the range 100 to 150°C. The reaction conditions employed allow the use of atmospheric pressure.

The mineral acid is preferably concentrated sulphuric acid as this gives rise to better yields of the ester of formula I.

It has been found necessary to employ substantially anhydrous conditions, that is to say, conditions under which the amount of water present during the reaction is kept to a minimum for example, below about 5% by weight of the reaction mixture, preferably below 1% and suitably below 0.5% by weight of the reaction mixture. For example, the reaction will tolerate the water normally present in concentrated sulphuric acid (2% by weight) at the concentrations of acid employed in the process, provided that the alcohol and the nitrile reactants are substantially dry.

As the reaction proceeds under the influence of the mineral acid catalyst, ammonia is generated thereby neutralizing the acid catalyst and thus sufficient quantities of the acid must be present in order to provide the necessary catalytic action. In general terms, the amount of mineral acid (based on the nitrile present in the process according to the invention) should lie in the range of from 3:1 to 1:1 by weight.

In the compounds of general formula II, $R^1$ and $R^2$ preferably represent methyl groups; $X_1$ and $X_2$ represent chlorine or bromine atoms or methyl groups; or $X^1$ represents a hydrogen atom and $X^2$ a 2,2-dichlorovinyl group, a 2,2-dibromovinyl group, a 2,2-difluorovinyl group, a 2-chloro-2-fluorovinyl group, or a 2-methyl-1-propenyl group; and R represents a 2-methoxyethyl group.

As has been stated hereinbefore, the alcoholysis reaction enables a cyclopropanecarbonitrile to be converted into the corresponding 2-methoxyethyl or 2-ethoxyethyl ester. Simultaneously, some 3,6-dioxaheptyl (when methoxyethanol has been used) or 3,6-dioxaoctyl (when ethoxyethanol has been used) ester of the corresponding substituted cyclopropanecarboxylic acid is formed (these esters are novel compounds), together with a very small amount of an unidentified ester of the corresponding substituted cyclopropanecarboxylic acid. The selectivity to the 2-methoxyethyl ester or the 2-ethoxyethyl ester is usually higher than 80% and the total of the selectivities to the three esters formed is usually higher than 95%. The expression "selectivity to a certain compound", given in a percentage, is defined as:

$$\frac{a}{b} \times 100,$$

wherein

a is the amount of the starting nitrile converted into that certain compound and b is the amount of the converted starting nitrile.

The ester of formula I may be isolated from the reaction mixture obtained by addition of water, extraction of the product obtained with an organic solvent, washing of the organic extract phase with alkaline-reacting water and evaporation of the solvent from the washed organic phase. The residue thus obtained also contains the 3,6-dioxaheptyl ester or the 3,6-dioxaoctyl ester, the unidentified ester mentioned hereinbefore and 2,5,8-trioxanonane (the ether derived from methoxyethanol) or 3,6,9-trioxaundecane (the ether derived from ethoxyethanol). The three esters present in the residue may then be converted to a "synthetic pyrethroid" via the free acid or the acid chloride by reaction with 3-phenoxybenzyl alcohol or *alpha*-cyano-3-phenoxybenzyl alcohol. The following nitriles are the most important in that they are intermediates for the pyrethroids of greatest pesticidal activity:—

2,2,3,3-tetramethylcyclopropanecarbonitrile;

2,2,-dimethyl-3-(2-methyl-1-propenyl)cyclopropanecarbonitrile;

3-(2,2-dichlorovinyl)-2,2-dimethylcyclopropanecarbonitrile;

3-(2,2-dibromovinyl)-2,2-dimethylcyclopropanecarbonitrile.

The novel esters of substituted cyclopropanecarboxylic acids referred to hereinbefore are esters of the general formula:—

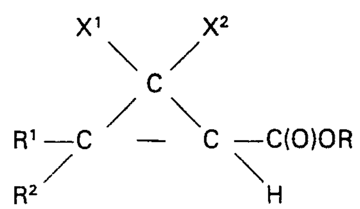

(I)

wherein

each of $R^1$ and $R^2$ independently represents an alkyl group of 1 to 4 carbon atoms or $R^1$ and $R^2$

together represent an alkylene group of up to 5 carbon atoms, other than methylene, each of $X^1$ and $X^2$ independently represents a hydrogen atom, an alkyl group of 1 to 4 carbon atoms, a halogen atom, or $X^1$ and $X^2$ together represent an alkylene group of up to 5 carbon atoms, other than methylene, or $X^1$ represents a hydrogen atom and $X^2$ a 2-halovinyl group, a 2,2-dihalovinyl group or a 2-methyl-1-propenyl group and R represents a 2-methoxyethyl or a 2-ethoxyethyl group.

2-Methoxyethyl 3-(2,2-dichlorovinyl)-2,2-dimethylcyclopropanecarboxylate is the preferred ester.

The invention is further illustrated by reference to the following Example.

## Example

Preparation of 2-methoxyethyl 3-(2,2-dichlorovinyl)-2,2-dimethylcyclopropanecarboxylate

A mixture obtained by combining 5 mmol. of substantially dry 3-(2,2-dichlorovinyl)-2,2-dimethylcyclopropanecarbonitrile and 12.5 ml of a sulphuric acid/2-methoxyethanol solution (20% of 98%w sulphuric acid in dried 2-methoxyethanol) was refluxed at 130°C (1 atmosphere) for 5.5 hours. After cooling to ambient temperature water (50 ml) was added and the product extracted with three 30-ml portions of methylene chloride. The organic phase was washed with 20 ml of an aqueous solution of sodium carbonate (concentration 0.5 mol. $Na_2CO_3/l$), dried over anhydrous magnesium sulphate and evaporated at a pressure of 14 mm Hg to give 1.2 g of a residue containing the following esters of 3-(2,2-dichlorovinyl)-2,2-dimethylcyclopropanecarboxylic acid, the selectivities to which were as indicated.

| Ester | Selectivity, % |
|---|---|
| the 2-methoxyethyl ester | 86 |
| the 3,6-dioxaheptyl ester | 9 |
| an unidentified ester | 2 |
| Total of the three esters | 97 |

The conversion of the starting nitrile was 96%.

The NMR spectrum of the title ester showed the following absorptions relative to a tetramethylsilane standard (measured at 90 MHz using a solution of the compound in deuterochloroform):

$\delta = 1.2$ ppm ($C\mathit{H}_3-C$)

$\delta = 4.2$ ppm ($-C(=O)-O-C\mathit{H}_2-CH_2-$)

$\delta = 1.6 - 2.4$ ppm (2*H*-atoms attached to the ring)

$\delta = 3.6$ ppm ($-C(=O)-O-CH_2-C\mathit{H}_2-$)

$\delta = 6.3$ ppm (cis $\mathit{H}-C=$)

$\delta = 3.3$ ppm ($-O-C\mathit{H}_3$)

$\delta = 5.6$ ppm (trans $\mathit{H}-C=$)

## Comparative Experiment

Preparation of 2-hydroxyethyl 3-(2,2-dichlorovinyl)-2,2-dimethylcyclopropanecarboxylate

A mixture obtained by combining 5 mmol, of substantially dry 3-(2,2-dichlorovinyl)-2,2-dimethylcyclopropanecarbonitrile and 12.5 ml of a sulphuric acid/ethylene glycol solution (10% by weight of 98%w sulphuric acid in dry glycol) was stirred at 120°C (1 atmosphere) for four hours. After cooling to ambient temperature, water (50 ml) was added and the product extracted with three 30 ml-portions of methylene chloride. The organic phase was washed with 20 ml of an aqueous solution of sodium carbonate (concentration 0.5 mol. $Na_2CO_3/l$), dried over anhydrous magnesium sulphate and evaporated at a pressure of 14 mm Hg to give 1.05 g of a residue containing the title ester in a yield of 63%, calculated on starting nitrile.

**Claims**

1. Process for the preparation of an ester having the general formula:

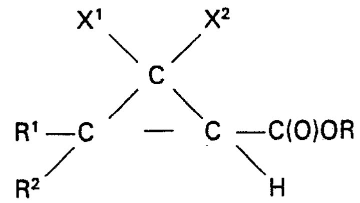

(I)

wherein

each of $R^1$ and $R^2$ independently represents an alkyl group of 1 to 4 carbon atoms or $R^1$ and $R^2$ together represent an alkylene group of up to 5 carbon atoms, other than methylene, each of $X^1$ and $X^2$ independently represents a hydrogen atom, an alkyl group of 1 to 4 carbon atoms, a halogen atom, or $X^1$ and $X^2$ together represent an alkylene group of up to 5 carbon atoms, other than methylene, or $X^1$ represents a hydrogen atom and $X^2$ a 2-halovinyl group, a 2,2-dihalovinyl group or a 2-methyl-1-propenyl group and R represents a 2-methoxyethyl or a 2-ethoxyethyl group, characterized in that a substituted cyclopropanecarbonitrile having the general formula:

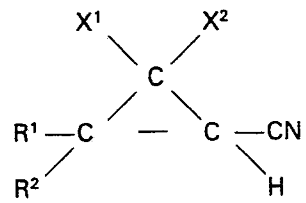

(II)

wherein

$R^1$, $R^2$, $X^1$ and $X^2$ have the same meaning as in formula I, is reacted with 2-methoxyethanol or 2-ethoxyethanol under substantially anhydrous conditions and in the presence of mineral acid acting as reaction catalyst and as scavenger for ammonia produced during the reaction.

2. Process according to claim 1, characterized in that it is carried out at a temperature in the range 80° to 200°C.

3. Process according to claim 1 or 2, characterized in that the mineral acid is concentrated sulphuric acid.

4. Process according to any one of the preceding claims, characterized in that the amount of mineral acid (based on nitrile) lies in the weight range 3:1 to 1:1.

5. Process according to any one of the preceding claims, characterized in that in formulae I and II, $R^1$ and $R^2$ represent methyl groups; $X^1$ and $X^2$ represent chlorine or bromine atoms or methyl groups; or $X^1$ represents a hydrogen atom and $X^2$ a 2,2-dichlorovinyl group, a 2,2-dibromovinyl group, a 2,2-difluorovinyl group, a 2-chloro-2-fluorovinyl group, or a 2-methyl-1-propenyl group; and R represents a 2-methoxyethyl group.

6. Process according to any one of the preceding claims, characterized in that the cyclopropanecarbonitrile is 3-(2,2-dichlorovinyl)-2,2-dimethylcyclopropanecarbonitrile.

7. Esters of the general formula:

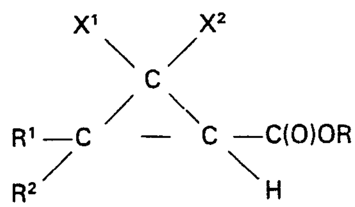

(I)

wherein

each of $R^1$ and $R^2$ independently represents an alkyl group of 1 to 4 carbon atoms or $R^1$ and $R^2$ together represent an alkylene group of up to 5 carbon atoms, other than methylene, each of $X^1$ and $X^2$ independently represents a hydrogen atom, an alkyl group of 1 to 4 carbon atoms, a halogen atom, or $X^1$ and $X^2$ together represent an alkylene group of up to 5 carbon atoms, other than methylene or $X^1$ represents a hydrogen atom and $X^2$ a 2-halovinyl group, a 2,2-dihalovinyl group or a 2-methyl-1-propenyl group and R represents a 2-methoxyethyl or a 2-ethoxyethyl group.

8. 2-Methoxyethyl 3-(2,2-dichlorovinyl)-2,2-dimethylcyclopropanecarboxylate.

9. 3,6-Dioxaheptyl 3-(2,2-dichlorovinyl)-2,2-dimethylcyclopropanecarboxylate.

**Revendications**

1. Procédé de préparation d'un ester ayant la formule générale:

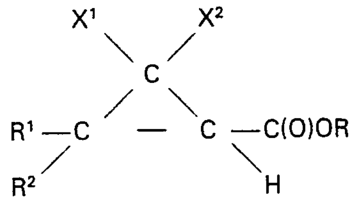

(I)

(où chacun des radicaux $R^1$ et $R^2$ représente de façon indépendante un groupe alcoyle ayant de 1 à 4 atomes de carbone, ou bien où $R^1$ et $R^2$ représentent ensemble un groupe alcoylène ayant jusqu'à 5 atomes de carbone, autre que le méthylène, chacun des radicaux $X^1$ et $X^2$ représentant de façon indépendante un atome d'hydrogène, un groupe alcoyle ayant de 1 à 4 atomes de carbone, un atome d'halogène, ou bien où $X^1$ et $X^2$ représentent ensemble un groupe alcoylène ayant jusqu'à 5 atomes de carbone, autre que le méthylène, ou bien où $X^1$ représente un atome d'hydrogène et $X^2$ un groupe 2-halovinyle, un groupe 2,2-dihalovinyle ou un groupe 2-méthyl-1-propényle, et R représente un groupe 2-méthoxyéthyle ou 2-éthoxyéthyle), caractérisé en ce qu'on fait réagir un cyclopropanecarbonitrile substitué de formule générale:

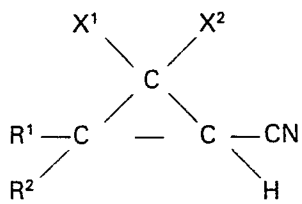

(II)

où $R^1$, $R^2$, $X^1$ et $X^2$ ont la même signification que dans la formule I, avec du 2-méthoxyéthanol ou du 2-éthoxyethanol dans des conditions pratiquement anhydres et en présence d'un acide minéral agissant comme catalyseur de la réaction et comme épurateur de l'ammoniac produit au cours de la réaction.

2. Procédé selon la revendication 1, caractérisé en ce qu'il se déroule à une température comprise dans un intervalle de 80° à 200°C.

3. Procédé selon les revendications 1 ou 2, caractérisé en ce que l'acide minéral est l'acide sulfurique concentré.

4. Procédé selon l'une quelconque des revendications précédentes, caractérisé en ce que la quantité d'acide minéral (fondée sur le nitrile) se situe dans un intervalle pondéral de 3:1 à 1:1.

5. Procédé selon l'une quelconque des revendications précédentes, caractérisé en ce que dans les formules I et II, $R^1$ et $R^2$ représentent des groupes méthyle; $X^1$ et $X^2$ représentent des atomes de chlore ou de brome ou des groupes méthyle; ou $X^1$ représente un atome d'hydrogène et $X^2$ un groupe 2,2-dichlorovinyle, un groupe 2,2-dibromovinyle, un groupe 2,2-difluorovinyle, un groupe 2-chloro-2-fluorovinyle, ou un groupe 2-méthyl-1-propényle; et R représente un groupe 2-méthoxyéthyle.

6. Procédé selon l'une quelconque des revendications précédentes, caractérisé en ce que le cyclopropanecarbonitrile est le 3-(2,2-dichlorovinyl)-2,2-diméthylcyclopropanecarbonitrile.

7. Esters de formule générale

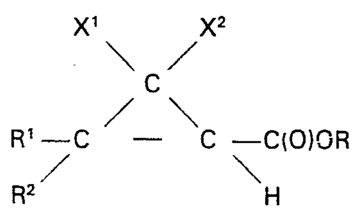

(I)

(où chacun des radicaux $R^1$ et $R^2$ représente de façon indépendante un groupe alcoyle ayant de 1 à 4 atomes de carbone, ou bien où $R^1$ et $R^2$ représentent ensemble un groupe alcoylène ayant jusqu'à 5 atomes de carbone, autre que le méthylène, chacun des radicaux $X^1$ et $X^2$ représentant de façon indépendante un atome d'hydrogène, un groupe alcoyle ayant de 1 à 4 atomes de carbone, un atome d'halogène, ou bien $X^1$ et $X^2$ représentent ensemble un groupe alcoylène ayant jusqu'à 5 atomes de carbone, autre que le méthylène, ou bien où $X^1$ représente un atome d'hydrogène et $X^2$ un groupe 2-halovinyle, un groupe 2,2-dihalovinyle ou un groupe 2-méthyl-1-propényle, et R représente un groupe 2-méthoxyéthyle ou 2-éthoxyéthyle).

8. 2-méthoxyéthyl-3-(2,2-dichlorovinyl)-2,2-diméthyl-cyclopropanecarboxylate.
9. 3,6-dioxaheptyl-3-(2,2-dichlorovinyl)-2,2-diméthyl-cyclopropanecarboxylate.

**Patentansprüche**

1. Verfahren zur Herstellung eines Esters der allgemeinen Formel:

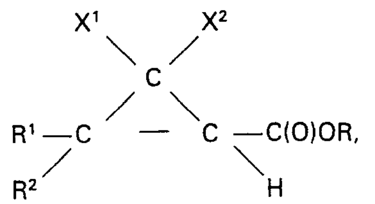

(I)

in der $R^1$ und $R^2$ unabhängig voneinander jeweils für eine Alkylgruppe mit 1 bis 4 Kohlenstoffatomen stehen, oder $R^1$ und $R^2$ zusammen eine Alkylengruppe mit bis zu 5 Kohlenstoffatomen ausgenommen die Methylengruppe bedeuten, $X^1$ und $X^2$ unabhängig voneinander jeweils für ein Wasserstoffatom, eine Alkylgruppe mit 1 bis 4 Kohlenstoffatomen oder 1 Halogenatom stehen oder $X^1$ und $X^2$ zusammen eine Alkylengruppe mit bis zu 5 Kohlenstoffatomen ausgenommen die Methylengruppe bedeuten oder $X^1$ für Wasserstoffatom steht und $X^2$ für eine 2-Halogenvinylgruppe, eine 2,2-Dihalogenvinylgruppe oder 2-Methyl-1-propenylgruppe und R eine 2-Methoxyäthyl- oder 2-Äthoxyäthylgruppe ist, dadurch gekennzeichnet, dass ein substituiertes Cyclopropancarbonsäurenitril der allgemeinen Formel:

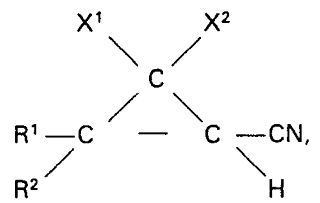

(II)

in der $R^1$, $R^2$, $X^1$ und $X^2$ die gleiche Bedeutung wie in Formel (I) haben, umgesetzt wird mit 2-Methoxyäthanol oder 2-Äthoxyäthanol unter im wesentlichen wasserfreien Bedingungen und in Gegenwart von Mineralsäure, die als Reaktionskatalysator wirkt, sowie eines Abfängers für im Verlauf der Reaktion entstandenen Ammoniak.

2. Verfahren nach Anspruch 1, dadurch gekennzeichnet, dass es bei einer Temperatur im Bereich 80 bis 200°C ausgeführt wird.

3. Verfahren nach Anspruch 1 oder 2, dadurch gekennzeichnet, dass die Mineralsäure konzentrierte Schwefelsäure ist.

4. Verfahren nach einem der vorangehenden Ansprüche, dadurch gekennzeichnet, dass die Menge an Mineralsäure (bezogen auf Nitril) im Gewichtsbereich 3:1 bis 1:1 liegt.

5. Verfahren nach einem der vorangehenden Ansprüche, dadurch gekennzeichnet, dass in den Formeln (I) und (II) $R^1$ und $R^2$ Methylgruppen darstellen, $X^1$ und $X^2$ für Chloratome oder Bromatome oder Methylgruppen stehen oder $X^1$ ein Wasserstoffatom ist und $X^2$ eine 2,2-Dichlorvinylgruppe, eine 2,2-Dibromvinylgruppe, eine 2,2-Difluorvinylgruppe, eine 2-Chlor-2-fluorvinylgruppe oder eine 2-Methyl-1-propenylgruppe und R eine 2-Methoxyäthylgruppe bedeutet.

6. Verfahren nach einem der vorangehenden Ansprüche, dadurch gekennzeichnet, dass das Cyclopropancarbonsäurenitril 3-(2,2-Dichlorvinyl)-2,2-dimethylcyclopropancarbonsäurenitril ist.

7. Ester der allgemeinen Formel:

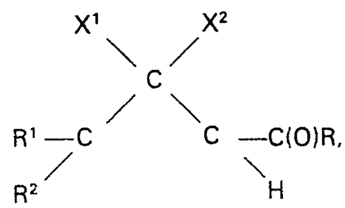

(I)

in der $R^1$ und $R^2$ unabhängig voneinander jeweils für eine Alkylgruppe mit 1 bis 4 Kohlenstoffatomen stehen, oder $R^1$ und $R^2$ zusammen eine Alkylengruppe mit bis zu 5 Kohlenstoffatomen ausgenommen die Methylengruppe bedeuten, $X^1$ und $X^2$ unabhängig voneinander jeweils eine Wasserstofatom, eine Alkylgruppe mit 1 bis 4 Kohlenstoffatomen, oder ein Halogenatom sind, oder $X^1$ und $X^2$ zusammen eine Alkylengruppe mit bis zu 5 Kohlenstoffatomen ausgenommen die Methylengruppe bedeuten, oder $X^1$ ein Wasserstoffatom ist und $X^2$ eine 2-Halogenvinylgruppe, eine 2,2-Dihalogenvinylgruppe oder eine 2-Methyl-1-propenylgruppe ist und R eine 2-Methoxyäthyl- oder 2-Äthoxyäthylgruppe bedeutet.

8. 2-Methoxyäthyl-3-(2,2-dichlorvinyl)-2,2-dimethyl-cyclopropancarboxylat.

9. 3,6-Dioxaheptyl-3-(2,2-dichlorvinyl)-2,2-dimethylcyclopropancarboxylat.